# EUROPEAN PATENT APPLICATION

(11) **EP 1 671 594 A1**
(43) Date of publication of application: **21.06.2006**
(21) Application number: 04792012.9
(22) Date of filing: 04.10.2004
(51) Int. Cl.: A61B 17/22

(54) **MEDICAL CLIPPING DEVICE, MEDICAL INSTRUMENT AND METHOD FOR CUTTING ORGANISM TISSUE UTILIZING THE DEVICE**

(30) Priority: 06.10.2003 JP 2003347310
(71) Applicant: Olympus Corporation, Tokyo 151-0072 (JP)
(72) Inventor: SEKINE, Ryuta, Int. Prop. Support Dept., Hachioji-shi, Tokyo 192-8512 (JP); SUZUKI, Akira Int. Prop. Support Dept., Hachioji-shi, Tokyo 192-8512 (JP); MATSUI, Raifu Intellectual Prop. Support Dept., Hachioji-shi, Tokyo 192-8512 (JP); OKADA, Yuta Intellectual Prop. Support Dept., Hachioji-shi, Tokyo 192-8512 (JP); NAKAHASHI, Kensei Intellectual Prop. Support Dept., Hachioji-shi, Tokyo 192-8512 (JP)
(74) Representative: von Hellfeld, Axel
(86) International application number: PCT/JP2004/014601
(87) International publication number: WO 2005/032384

(57) **Abstract**

When a living tissue (H) of a ligation target is ligated by means of a snare wire (5), the snare wire (5) is brought into contact with the living tissue (H) of a ligation target, on a large contact face by means for preventing living tissue cut-in of a ligation target of at least one of a distal end chip (23) and a rear end ring (24) of a loop section (4) of the snare wire (5), thereby preventing the living tissue (H) of a ligation target from cutting into a contact face of at least one of the distal end chip (23) and rear end ring (24) of the loop section (4).

## Description

### Technical Field

The present invention relates to a medical ligation apparatus which is a surgical instrument used to be inserted in a body to retain a ligation target in the body in a state in which ligation is established by a detention snare; and a surgical instrument and method for living tissue resection utilizing the apparatus.

### Background Art

In general, it is widely known to insert a surgical instrument into a body through an inside of a channel of an endoscope inserted in advance into the body, and then, treat a lesion or the like of a living tissue in the body. The following technique has been conventionally developed as a system for carrying out surgery using an endoscope of this type. That is, first, a surgical instrument is inserted into a body through a channel of an endoscope, and a root portion of a polyp shaped living tissue formed in the body is throttled and ligated by a detention snare or T-BAR and the like. Then, an upper portion than the ligated site of the polyp shaped living tissue is resected by a resection surgical instrument such as a resection snare. In this manner, this system carries out resection of the living tissue without unexpected bleeding or perforation.

In Jpn. Pat. Appln. KOKAI Publication No. 10-277046 or 11-244294, there is disclosed an example of a detention snare used as a ligation instrument for ligating a root portion of a living tissue. A snare wire is provided at this detention snare. A ligation loop section which is expandable in a loop shape is formed at a distal end side of the snare wire. A tubular throttle member is provided at a proximal end side of the loop section. Two snare wires at both ends of the snare wire which produces the loop section are press-fitted to this throttle member in a state in which the snare wires are bundled. Further, a coupling ring is provided at a proximal end side of the snare wire.

In addition, the detention snare is manipulated by means of a snare manipulating instrument. This snare manipulating instrument has: an elongated insert section inserted into a body; and a manipulating section at hand side coupled to a proximal end of the insert section. The insert section has: an outer sheath; a manipulating tube inserted into this outer sheath so as to be movable in an axial direction; and a manipulating wire inserted into this manipulating tube so as to be movable in an axial direction. A snare coupling section is provided at a distal end of the manipulating wire. The coupling ring is removably coupled with the snare coupling section. The manipulating section has a manipulating tube slider and a manipulating wire slider. The manipulating tube slider moves and manipulates a manipulating tube in an axial direction. The manipulating wire slider moves and manipulates the manipulating wire in an axial direction.

Then, when the detention snare is used, the coupling ring of the detention snare is coupled in advance with the snare coupling section of the manipulating wire. In this state, the manipulating wire is manipulated to be backwardly pulled, and is set so that the entire detention snare is housed in the outer sheath. Then, the insert section of the snare manipulating instrument is inserted into a body through an inside of a channel of an endoscope.

Further, at a proximal position of a living tissue targeted for surgery, the detention snare is extended to the outside of the outer sheath. At this time, the loop section of the detention snare is expanded in a loop shape by means of elasticity of the snare wire itself. Then, a manipulation of inserting a living tissue of a ligation target into the loop section of this snare wire is carried out. In a state in which the living tissue of a ligation target has been inserted, the manipulating tube of the snare manipulating instrument is manipulated to be forwardly pushed out. At this time, the throttle member is manipulated to be pushed out at the distal end side of the snare wire by means of the manipulating tube, whereby the living tissue of a ligation target in the loop section of the snare wire is ligated by manipulating the loop section of the snare wire to be throttled in a throttling direction.

In addition, after a ligation target has been ligated by the detention snare, the coupling ring of the detention snare is pulled out from the snare coupling section at a distal end of the manipulating wire, and the detention snare is separated from the snare manipulating instrument. Further, after the root portion of the polyp shaped living tissue has been ligated by the detention snare, an upper portion of the ligated portion is resected by means of a resection surgical instrument such as a resection snare as required.

### Disclosure of Invention

The above detention snare having a conventional configuration ligates a root portion of a living tissue by means of a thin snare wire. In this case, the thin snare wire easily strongly cuts into the living tissue when a throttle quantity of the ligated portion is increased and a diameter of the ligated portion is decreased. Thus, for example, there is a possibility that a blood flow in the inside of the ligated portion is inhibited.

In addition, in a conventional living tissue resection system, when the upper portion of the ligated site ligated by means of the thin snare wire of the detention snare is resected by means of a resection snare, there is a possibility that the resection snare slips from the upper portion of the ligated site of the living tissue to the root side while throttling is carried out by the detention snare. In such a case, a position of a resection face of the living tissue by the resection snare moves up to the vicinity immediately above the detention snare. Thus, it may be impossible to provide a sufficient margin between a throttle position caused by the thin snare wire of the detention snare and a resection face of the living tissue caused by the resection snare.

The present invention has been made in view of the above-described circumstance. It is an object of the present invention to provide a medical ligation apparatus capable of: preventing a snare wire from cutting into a living tissue when ligating the living tissue by a detention snare; reliably resecting, with a resection surgical instrument, an upper portion of a ligated site of the living tissue ligated by means of the detention snare with a margin; and easily carrying out the resection work, and a surgical instrument and method for living tissue resection utilizing the apparatus.

According to a first aspect of the present invention, there is provided a medical ligation apparatus comprising:
a detention snare inserted into a body and retained therein; and
a snare manipulating instrument which manipulates the detention snare,
the detention snare having:
   a snare wire having a distal end and a proximal end, the snare wire having formed thereat a ligation loop section which is expandable in a loop shape at the distal end side thereof;
   a coupling ring provided at a proximal end side of the snare wire; and
   a ring shaped throttle member into which the proximal end side of the snare wire is inserted in a press-fitted state,
   the snare manipulating instrument having:
      an elongated insert section having a distal end and a proximal end and inserted into a body; and
      a snare coupling section provided at a distal end of the insert section, the snare coupling section having the coupling ring removably coupled therewith,
wherein the medical ligation apparatus comprises:
ligation means for ligating a ligation target in a loop section of the snare wire by manipulating the loop section of the snare wire to be throttled in a throttle direction by manipulating the throttle member to be pushed out to a distal end side of the snare wire in a state in which the detention snare is coupled with the snare coupling section and a living tissue of a ligation target is inserted into the loop section of the snare wire; and
living tissue cut-in preventing means which is disposed in at least one of a distal end side and a rear end side of the loop section of the snare wire, and when the living tissue of the ligation target is ligated by means of the snare wire, the means comprising a contact face on which a contact area with the living tissue is greater than a contact area of the snare wire and the living tissue.

In addition, in the present invention, when a living tissue of a ligation target is ligated by means of a snare wire, the snare wire is brought into contact with the living tissue of a ligation target, on a large contact face by means for preventing living tissue cut-in of a ligation target of at least one of a distal end side and a rear end side of a loop section of the snare wire, thereby preventing the living tissue of a ligation target from cutting into a contact face of at least one of the distal end side and proximal end side of the loop section. Further, a resection surgical instrument such as a resection snare is positioned by cut-in preventing means at the upper portion of the ligated site of the living tissue ligated by means of the detention snare, whereby a sufficient margin is provided between a resection face of the living tissue using a resection surgical instrument such as the resection snare and a loop section of the snare wire.

The living tissue cut-in preventing means may have slip preventing means with a living tissue of a ligation target on the contact face.

The living tissue of a ligation target is prevented from slipping from a contact face with cut-in preventing means by slip-proof means of the living tissue cut-in preventing means.

The living tissue cut-in preventing means may be provided forwardly and backwardly of a loop section of the snare wire, respectively.

When the living tissue of a ligation target is ligated by means of the snare wire, the snare wire is brought into contact with the living tissue of a ligation target on a large contact face by living tissue cut-in preventing means before or after the loop section of the snare wire, thereby preventing the living tissue of a ligation target from cutting into the contact face before or after the loop section. Further, the resection surgical instrument such as the resection snare is positioned by cut-in preventing means at the upper portion of the ligated site of the living tissue ligated by means of the detention snare, whereby a sufficient margin can be provided between a resection face of the living tissue by the resection surgical instrument such as the resection snare and the loop section of the snare wire.

The living tissue cut-in preventing means may have a cutaway setting section for, when resecting an upper side of a ligated site at a root side of a living tissue of a ligation target ligated by the detention snare, setting a predetermined cutaway between a resection face at the upper side of the ligated site and the ligated site.

When resecting the upper side of the ligated site at the root section of the living tissue of a ligation target ligated by means of the detention snare, a predetermined cutaway is set between a ligated resection site and an upper resection face thereof, by means of a cutaway setting section of the living tissue cut-in preventing means.

The cutaway setting section may be formed of a clip member mounted on the ligated site of the living tissue, after the living tissue is ligated by throttling the loop section by the throttle member.

When the living tissue of a ligation target is ligated by means of the snare wire, after the living tissue has been ligated by throttling the loop section of the snare wire by a throttle member, a clip member independent of the throttle member is mounted on a ligated site of the living tissue, and a resection surgical instrument such as a resection snare is positioned by means of the clip member of the cut-in preventing means at the upper portion of the ligated site of the living tissue ligated by means of the detention snare. In this manner, a sufficient margin can be provided between the resection face of the living tissue by the resection surgical instrument such as the resection snare and the loop section of the snare wire.

According to a second aspect of the present invention, there is provided a surgical instrument comprising:
a surgery insert assisting instrument comprising:
   an opening section having formed at an insert section inserted into a cavity in a body at least an endoscope insert channel, a ligation channel into which a ligation surgical instrument for inserting a ligation instrument for ligation of a living tissue is inserted, and a resection channel into which a resection surgical instrument for resecting a ligated site of the living tissue ligated by the ligation instrument is inserted, the opening section being disposed at a distal end of the insert section to insert the living tissue of the ligation target into one side of a distal end outer periphery face of the insert section; and a work space section formed at the other end of the distal end outer periphery face to manipulate the living tissue of the ligation target to be pulled up from the opening section;
   the ligation surgical instrument inserted into the ligation channel and set to be disposed at a position surrounding the periphery of the opening section;
   an endoscope inserted into the work space section through the endoscope insert channel;
   a grip surgical instrument having provided thereat a grip section which grips the living tissue at a distal end of an insert section inserted into the work space section through the endoscope insert channel;
   tissue pulling up means for manipulating the living tissue of the ligation target to be pulled up through an inside of the opening section by means of a curve manipulation of the endoscope in a state in which the living tissue is gripped by means of the grip section of the grip surgical instrument;
   ligation means for ligating by means of the ligation surgical instrument the living tissue of the ligation target pulled up by the tissue pulling up means; and
   a resection surgical instrument inserted into the resection channel, the resection surgical instrument resecting the ligated site of the living tissue ligated
   by the ligation means,
wherein a shape of the opening section of the surgery insert assisting instrument is set so as to reduce a length in a direction orthogonal to the axial direction as compared with a length in an axial direction of the surgery insert assisting instrument.

According to a third aspect of the present invention, there is provided a living tissue resection method, comprising:
an insertion step of, in a state in which a detention snare is removably coupled with a snare coupling section of a snare manipulating instrument, inserting the detention snare into a body;
a ligation step of, in a state in which a ligation target is inserted into a loop section of a snare wire of the detention snare, ligating the ligation target in the loop section of the snare wire by manipulating the loop section of the snare wire to be throttled in a throttle direction;
a detention step of, after ligating the living tissue of the ligation target, removing and retaining the detention snare from the snare coupling section of the snare manipulating instrument; and
a living tissue resection step of, after a resection surgical instrument has been disposed at an upper portion of a ligated site at a root site of the living tissue of the ligation target ligated by means of the detention snare, and in a state in which the resection surgical instrument is moved while a throttle member for throttling the loop section of the snare wire is used as a guide, and then, a predetermined cutaway is left between an upper resection face of the ligated site and the ligated site, resecting the upper portion of the ligated site.

### Brief Description of Drawings

FIG. 1 is a perspective view showing an appearance of a whole medical ligation apparatus according to a first embodiment of the present invention;
FIG. 2A is a perspective view showing a detention snare of the medical ligation apparatus according to the first embodiment;
FIG. 2B is a perspective view showing a distal end of a snare manipulating instrument;
FIG. 2C is a perspective view showing a manipulating section at the front face of the snare manipulating instrument;
FIG. 3 is a longitudinal cross section of essential portions showing a state in which a detention snare is coupled with the snare manipulating instrument of the medical ligation apparatus according to the first embodiment;
FIG. 4 is a perspective view of essential portions showing a state in which a living tissue is inserted into the detention snare of the medical ligation apparatus according to the first embodiment;
FIG. 5 is a perspective view of essential portions showing a state in which a detention snare is retained in a body in a state in which the living tissue is ligated by means of the detention snare of the medical ligation apparatus according to the first embodiment;
FIG. 6A a side view of essential portions showing a ligated site of the living tissue by means of the detention snare according to the first embodiment;
FIG. 6B is a sectional view taken along the line VIB-VIB of FIG. 6A;
FIG. 7 is an illustrative view adopted to illustrate a work of a resection snare resecting an upper portion of a ligated site of a living tissue by means of the detention snare according to the first embodiment;
FIG. 8 is an illustrative view adopted to illustrate a work of a resection snare resecting an upper portion of a ligated site of a living tissue by means of the detention snare according to the first embodiment;
FIG. 9 is a longitudinal cross section of essential portions of a detention snare showing a second embodiment of the present invention;
FIG. 10 is a longitudinal cross section of essential portions of a detention snare showing a third embodiment of the present invention;
FIG. 11 is a longitudinal cross section of essential portions of a detention snare showing a fourth embodiment of the present invention;
FIG. 12 is a longitudinal cross section of essential portions of a detention snare showing a fifth embodiment of the present invention;
FIG. 13A is a longitudinal cross section of essential portions showing a state in which a detention snare according to a sixth embodiment of the present invention is housed in an outer sheath of a snare manipulating instrument;
FIG. 13B is a longitudinal cross section of essential portions showing a state in which a detention snare is protruded outside of an outer sheath of a snare manipulating instrument;
FIG. 14 is a longitudinal cross section of essential portions showing a medical ligation apparatus according to a seventh embodiment of the present invention;
FIG. 15A is a perspective view of essential portions showing a state in which a living tissue is inserted into a detention snare of the medical ligation instrument according to the seventh embodiment;
FIG. 15B is a perspective view of essential portions showing a state in which a living tissue is ligated by means of a detention snare;
FIG. 15C is a perspective view of essential portions showing a state in which a clip member is mounted on a ligated site of a living tissue by means of a detention snare;
FIG. 16 is a perspective view showing a general configuration of a whole system of a medical ligation apparatus according to an eighth embodiment of the present invention;
FIG. 17 is a perspective view of essential portions of the medical ligation apparatus according to the eighth embodiment;
FIG. 18 is a longitudinal cross section of essential portions showing a coupling member of the medical ligation apparatus according to the eighth embodiment;
FIG. 19 is a perspective view showing a general configuration of a whole surgical instrument system for fully resecting a colon according to a ninth embodiment of the present invention;
FIG. 20A is a plan view showing a distal end portion of a surgery insert assisting instrument of the surgical instrument system according to the ninth embodiment;
FIG. 20B is a sectional view taken along the line 20B-20B of FIG. 20A;
FIG. 21 is a longitudinal cross section of essential portions showing a state in which a living tissue is pulled up from a surgery hole to a work space by means of the surgical instrument system according to the ninth embodiment;
FIG. 22 is a longitudinal cross section of essential portion showing a state in which the living tissue pulled up by means of the surgical instrument system according to the ninth embodiment is ligated by means of a ligation snare and a resection snare;
FIG. 23 is a longitudinal cross section of essential portions showing a state in which the living tissue pulled up by means of the surgical instrument system according to the ninth embodiment is resected by means of a resection snare;
FIG. 24A is a view sowing a resected portion of a colon resected by means of the surgical instrument system according to the ninth embodiment;
FIG. 24B is a view showing a state in which a resection portion of a colon is ligated by means of a detention snare;
FIG. 25A is a perspective view showing a detention snare according to a tenth embodiment of the present invention;
FIG. 25B is a longitudinal cross section of essential portions showing a state in which a living tissue is ligated by means of a detention snare;
FIG. 26A is a longitudinal cross section showing a distal end portion of a surgery insert assisting instrument of a surgical instrument system according to an eleventh embodiment of the present invention;
FIG. 26B is a sectional view taken along the line 26B-26B of FIG. 26A;
FIG. 26C is a longitudinal cross section of essential portions showing a state in which the living tissue pulled up by means of the surgical instrument system is ligated by means of a ligation instrument; and
FIG. 26D is a longitudinal cross section of essential portions showing a state in which the living tissue pulled up by means of the surgical instrument system is resected by means of a resection snare.

### Best Mode for Carrying Out the Invention

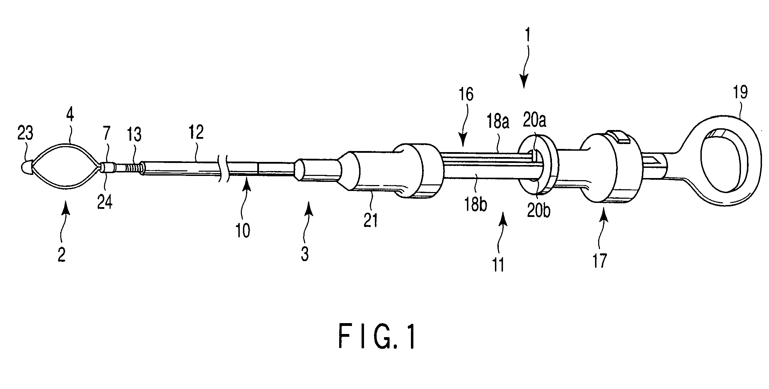

Hereinafter, a first embodiment of the present invention will be described with reference to FIGS. 1 to 8. FIG. 1 shows an appearance of a whole medical ligation apparatus 1 according to the present embodiment. This medical ligation apparatus 1 has a detention snare 2 which is a ligation instrument and a snare manipulating instrument 3 for manipulating this detention snare 2.

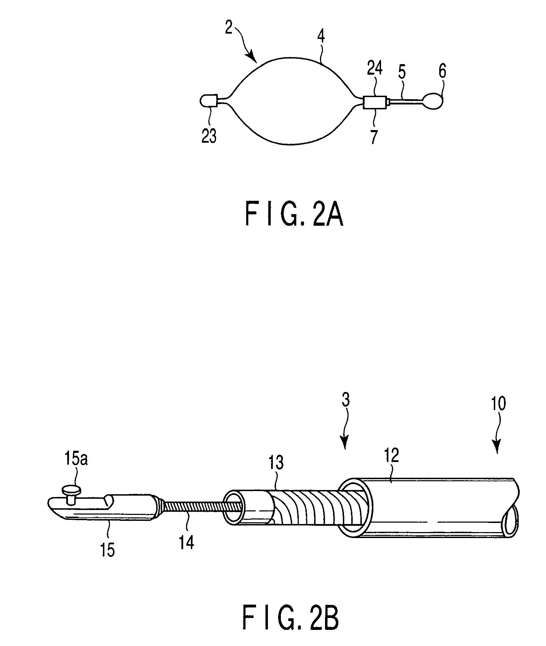

FIG. 2A shows the detention snare 2 according to the present embodiment. This detention snare 2 has: a snare wire 5 having formed thereat a ligation loop section 4 which is expandable in a loop at a distal end side; a coupling ring 6 provided at a proximal end of this snare wire 5; and a ring shaped throttle member 7 into which two wire portions (wire rods 5b, 5c) in the vicinity of an end of the coupling ring 6 of the snare wire 5 are inserted in a press-fit state. The snare wire 5 is formed of a synthetic resin material having a good biocompatibility.

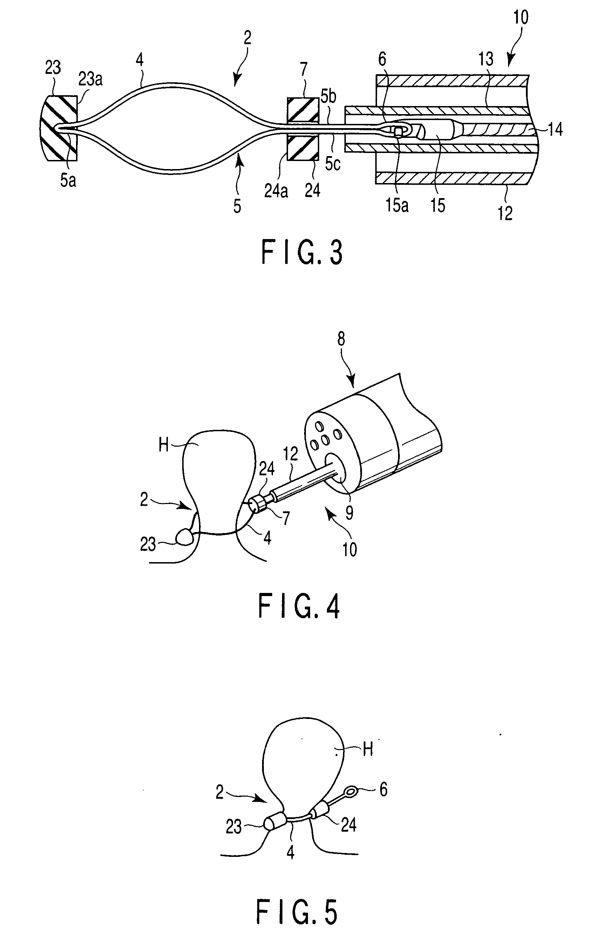

In addition, as shown in FIG. 3, in the detention snare 2, for example, the snare wire 5 is bent in a loop shape, and the loop section 4 is formed. Then, a fold section 5a of the snare wire 5 is disposed at a distal end side of the loop section 4. The two wire rods 5b, 5c of the snare wire 5 formed by being bent by the bent section 5a are extended to a proximal end side of the loop section 4. The two wire rods 5b, 5c extended to the proximal end side of the loop section 4 are press-fitted to the inside of the throttle member 7 in a bundled state. Further, the coupling ring 6 is disposed at a portion of an extension end of the two wire rods 5b, 5c extended backwardly of the throttle member 7.

As shown in FIG. 1, the snare manipulating instrument 3 has an elongated insert section 10 and a backwardly manipulating section 11. The manipulating section 11 is coupled with a proximal end of the insert section 10. As shown in FIG. 4, the insert section 10 is inserted into a body through the inside of a channel 9 of an endoscope 8. FIG. 4 shows a state in which the insert section 10 of the snare manipulating instrument 3 is inserted into the body through the inside of the channel 9 of the endoscope 8.

As shown in FIG. 2B, the insert section 10 has an outer sheath 12, a manipulating tube 13, and a manipulating wire 14. The manipulating tube 13 is inserted to be movable in an axial direction in the inside of the outer sheath 12. Further, the manipulating wire 14 is inserted to be movable in an axial direction in the inside of the manipulating tube 13.

A proximal end of a hook member (snare coupling section) 15 is connected and fixed to a distal end of the manipulating wire 14. A claw shaped hook 15a is provided at a distal end of this hook member 15. The hook 15a is protruded toward a direction substantially orthogonal to an axial direction of the manipulating wire 14. The coupling ring 6 of the detention snare 2 is removably engaged with this hook 15a.

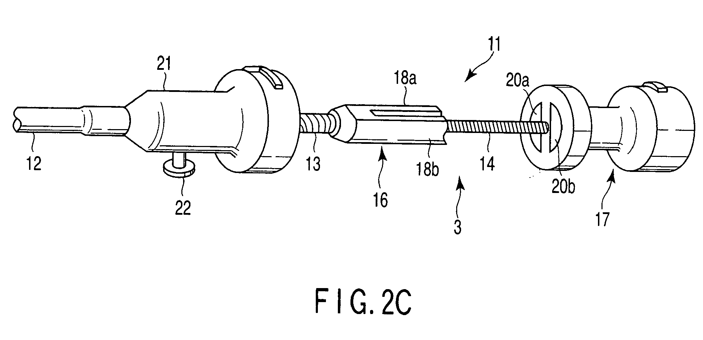

As shown in FIG. 2C, the manipulating section 11 has a manipulating section main body 16 and a slider section 17. The manipulating section main body 16 is extended toward an axial direction of the insert section 10. The slider section 17 is slidable in the axial direction of the insert section 10 along the manipulating section main body 16. A pair of guide rails 18a, 18b parallel to each other are provided at the manipulating section main body 16. A distal end of this manipulating section main body 16 is connected and fixed to a proximal end of the manipulating tube 13. Further, a finger hook ring 19 is assembled at a proximal end of the manipulating section main body 16. The finger hook ring 19 is rotatable in an axially turning direction of an axle of the manipulating section main body 16.

A pair of hole sections 20a, 20b are formed at the slider section 17. A pair of guide rails 18a, 18b of the manipulating section main body 16 are inserted into the hole sections 20a, 20b, respectively. Then, the slider section 17 is slidably supported in an axial direction of the insert section 10 in a state in which the slider section is guided by the guide rails 18a, 18b of the manipulating section main body 16.

Further, a rear end of the manipulating wire 14 is connected and fixed to the slider section 17. Then, the manipulating wire 14 is subjected to a push and pull manipulation with a slide operation of this slider section 17.

In addition, an outer sheath grip section 21 is coupled with a proximal end of the outer sheath 12. This outer sheath grip section 21 is retractably assembled in the axial direction of the insert section 10 at the distal end of the manipulating section main body 16. A cock section 22 is protruded on an outer periphery face of the outer sheath grip section 21.

Then, the snare manipulating instrument 3 is set in a state in which the hook 15a of the hook member 15 is inserted into the coupling ring 6 of the detention snare 2 and is removably engaged therewith. In this state, this manipulating instrument is used for manipulation of the detention snare 2.

In addition, as shown in FIG. 3, in the detention snare 2, a distal end chip (living tissue cut-in preventing means) 23 is fixed to the distal end of the loop section 4 of the snare wire 5. This distal end chip 23 comprises a contact face 23a on which a contact area of a living tissue H of a ligation target is greater than that of the snare wire 5. Further, the throttle member 7 is formed of a rear end ring (living tissue cut-in preventing means) 24 which comprises a contact area 24a on which the contact area of the living tissue H of a ligation target is greater than that of the snare wire 5 in the same manner as the distal end chip 23. The distal end chip 23 and the rear end ring 24 are formed of an elastic material such as a rubber, for example, or alternatively, a synthetic resin material such as plastic.

Now, a function of the above constituent elements will be described here. When using the medical ligation apparatus 1 according to the present embodiment, the detention snare 2 is set in advance to be coupled with the snare manipulating instrument 3. At the time of this coupling work, as shown in FIG. 2B, a distal end of the manipulating tube 13 is forwardly protruded from a distal end of the outer sheath 12 of the snare manipulating instrument 3. Then, the hook member 15 of the manipulating wire 14 is forwardly protruded from a distal end of the manipulating tube 13. In this state, the hook 15a of the hook member 15 is inserted into the coupling ring 6 of the detention snare 2, and then, is removably engaged therewith.

Then, the manipulating wire 14 is manipulated to be backwardly pulled. In this manner, a rear end of the throttle member 7 of the detention snare 2 is moved to a position at which the rear end lightly comes into pressure contact with a distal end of the manipulating tube 13. In this state, the whole detention snare 2 is moved to a position at which it is housed in the outer sheath 12. This state is mainly retained when the detention snare 2 is not used such as when the snare manipulating instrument 3 is inserted into the channel 9 of the endoscope 8.

A distal end of the insert section 10 of the snare manipulating instrument 3 inserted into a body through the inside of the channel 9 of the endoscope 8 is guided in a direction of the target living tissue H. At a time point when the distal end of the insert section 10 reaches a proximal position of the target living tissue H, the detention snare 2 is forwardly protruded from a distal end of the outer sheath 12 together with the manipulating tube 13 as shown in FIG. 1. At this time, the loop section 4 of the detention snare 2 is expanded in a loop shape, as shown in FIG. 2A by means of elasticity of the snare wire 5 itself.

Then, the snare manipulating instrument 3 is manipulated, and the target living tissue H is inserted into the loop section 4 of the detention snare 2, as shown in FIG. 4. In this state, the slider section 17 of the snare manipulating instrument 3 is manipulated to be pulled to the rear side of the manipulating section main body 16. At this time, the throttle member 7 is relatively pushed out forwardly (to the distal end side of the loop section 4) by means of the manipulating tube 13 together with the manipulating wire 14 being manipulated to be pulled. In this manner, the loop section 4 of the snare wire 5 is throttled. As a result, as shown in FIG. 5, a root section of the target living tissue H inserted into the loop section 4 is throttled by means of the loop section 4 of the snare wire 5.

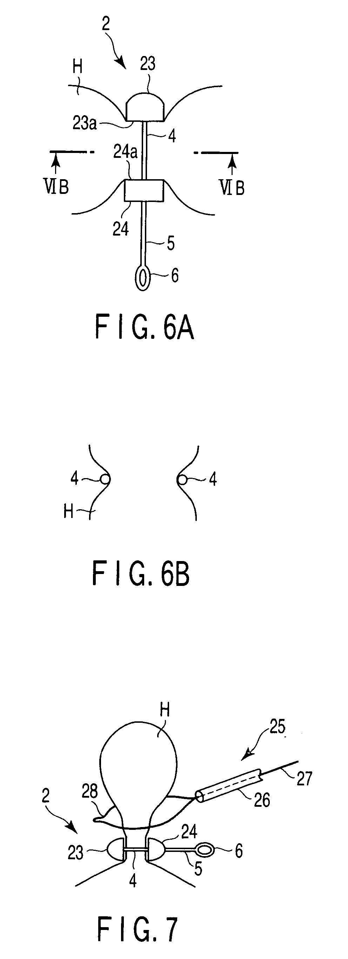

When the loop section 4 of this snare wire 5 is subjected to a throttle manipulation (when the living tissue H of a ligation target is ligated by means of the snare wire 5), as shown in FIG. 6A, the distal end chip 23 and rear end ring 24 before or after the loop section 4 of the snare wire 5 abut against the living tissue H of a ligation target in a planer contact state. At this time, in a region between the preceding and succeeding distal end chip 23 and the rear end ring 24, as shown in FIG. 6B, the snare wire 5 of the loop section 4 comes into direct contact with the living tissue H of a ligation target. Thus, at the time of throttle operation of the snare wire 5, at an abutment section before or after the loop section 4 at which a large force acts on the living tissue H of a ligation target, a contact face 23a of the distal end chip 23 whose contact area is wide and a contact face 24a of the rear end ring 24 come into contact with the living tissue H of a ligation target, respectively. In this manner, at the abutment section before or after the loop section 4 at which a large force acts at the time of throttle operation of the loop section 4, the large force acting on the living tissue H of a ligation target can be dispersed to the entire contact face 23a, 24a whose contact area is wide. As a result, the loop section 4 can be prevented from unnecessarily cutting into the living tissue H of a ligation target at the abutment section before or after the loop section 4.

Further, as shown in FIG. 6B, the loop section 4 situated between the distal end chip 23 and the rear end ring 24 abuts against the living tissue H laterally. A force acting on the living tissue H laterally is small as compared with the abutment section before or after the loop section 4 described previously, and thus, the snare wire 5 necessarily cuts into the living tissue H between the distal end chip 23 and the rear end ring 24, and fixes the detention snare 2.

Then, the hook 15a of the hook member 15 is pulled out from the coupling ring 6 of the detention snare 2. In this manner, as shown in FIG. 5, only the detention snare 2 is retained in a body in a state in which the root portion of the target living tissue H is ligated.

In addition, after the detention snare 2 has been retained, in the case where an upper portion of the ligated site of the ligated living tissue H is resected, a resection snare 25 serving as a resection surgical instrument is inserted into a body through the inside of the channel 9 of the endoscope 8, as shown in FIG. 7. This resection snare 25 has a resection snare introducing tube 26, an elongated manipulating wire 27, and a resection loop section 28. The manipulating wire 27 is inserted into the tube 26 so as to be movable in an axial direction. The loop section 28 is arranged at a distal end of the manipulating wire 27. Further, the loop section 28 is designed to expand in a loop shape.

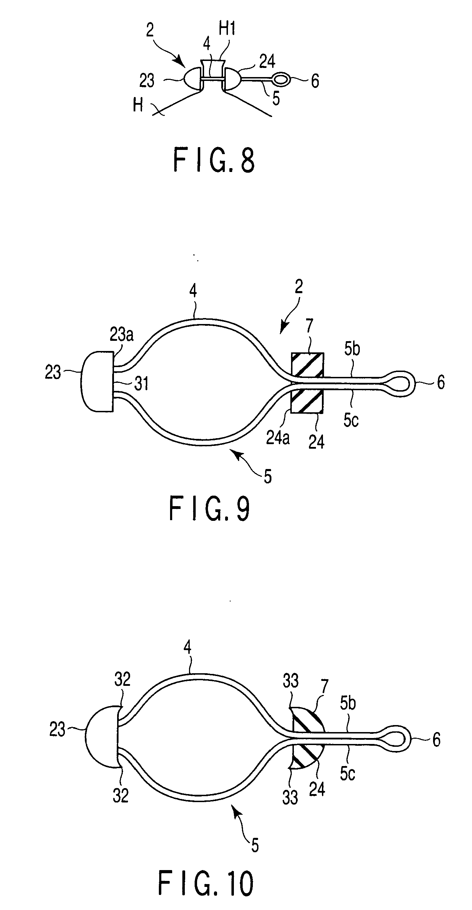

Then, as shown in FIG. 7, the target living tissue H is inserted into the loop section 28 of the resection snare 25. In this state, the resection snare introducing tube 26 is manipulated to be forwardly pushed out. At this time, the loop section 28 of the resection snare 25 is pulled into the resection snare introducing tube 26. In this manner, the upper portion of the ligated site of the living tissue H ligated by means of the detention snare 2 is resected by means of the loop section 28 of the resection snare 25. At this time, as shown in FIG. 8, a cut face H1 resected by means of the resection snare 25 is formed at the upper portion of the ligated site of the living tissue H ligated by means of the detention snare 2. Further, the cut piece of the resected living tissue H is collected (acquired) by either one of the following two methods. In a first method, for example, a grip forceps is inserted into a body through the inside of the channel 9 of the endoscope 8. In a state in which the cut piece is gripped by this grip forceps, the cut piece is taken out of the body and acquired together with the grip forceps. In a second method, a cut piece is suctioned out of the body through the inside of the channel 9 of the endoscope 8, and is acquired.

With the above configuration, the following advantageous effect is attained. That is, in the present embodiment, when the living tissue H is throttled by means of the detention snare 2, as shown in FIG. 6A, the distal end chip 23 before or after the loop section 4 of the snare wire 5 and the rear end ring 24 can be abutted against each other in a planer contact with the living tissue H of a ligation target. Thus, when the loop section 4 of the snare wire 5 is subjected to a throttle operation (when the living tissue H of a ligation target is ligated by means of the snare wire 5), at the abutment section before or after the loop section 4 at which a large force acts on the living tissue H of a ligation target, a force acting on the living tissue H of a ligation target can be dispersed to the whole contact faces 23a, 24a whose contact area is wide. Thus, at the abutment section before or after the loop section 4, the loop section 4 can be prevented from cutting into the living tissue H of a ligation target.

In addition, in a region between the distal end chip 23 and the rear end ring 24, a shown in FIG. 6B, the snare wire 5 of the loop section 4 is directly brought into contact with the living tissue H of a ligation target. Thus, this snare wire 5 can be cut into the living tissue H with a comparatively light force. Therefore, the detention snare 2 can be prevented from slipping off from the living tissue H of a ligation target. At this time, in a region between the distal end chip 23 and the rear end ring 24, the snare wire 5 can be cut into the living tissue H with a relatively light force. Thus, for example, there is no danger that a blood flow inside of the ligated site is inhibited.

Further, in the case of resecting the upper portion of the ligated site of the living tissue H ligated by means of the detention snare 2, the loop section 28 of the resection snare 25 is abutted against the distal end chip 23 of the detention snare 2 and an upper end of the rear end ring 24. In this manner, a position to be resected of the living tissue H can be positioned by means of the resection snare 25. Therefore, a sufficient margin (for example, about 1 mm to 5 mm) can be provided between the resected face H1 of the living tissue H caused by the resection snare 25 and the loop section 4 of the snare wire 5 of the detention snare 2. As a result, when the upper portion of the ligated site of the living tissue H ligated by means of the detention snare 2 is resected by means of the loop section 28 of the resection snare 25, the resection snare 25 can be reliably prevented from slipping off from the upper portion to the root side of the ligated site of the living tissue H.

Further, a front end face of the distal end chip 23 of the detention snare 2 and a rear end face of the rear end ring 24 may be configured to have roundness. In this manner, when the upper portion of the ligated site of the living tissue H ligated by means of the detention snare 2 is resected by means of the resection snare 25, the resection snare 25 can be smoothly guided to the resection face H1 of the living tissue H by means of a round portions of the front end face of the distal end chip 23 and the rear end face of the rear end ring 24. Thus, a work of resecting the resection face H1 of the living tissue H by means of the resection snare 25 can be easily carried out.

FIG. 9 shows a second embodiment of the present invention. The present embodiment changes a configuration of the detention snare 2 of the medical ligation apparatus 1 according to the first embodiment (refer to FIGS. 1 to 8) as follows.

That is, in a detention snare 2 according to the present embodiment, a wire gap retaining section 31 is provided at a coupling section between a rear end face of a distal end chip 23 and a loop section 4 of a snare wire 5. The wire gap retaining section 31 is retained in s state in which two wire rods 5b, 5c at a front end of the loop section 4 of the snare wire 5 are spaced from each other.

Then, in the detention snare 2 according to the present embodiment, when the loop section 4 of the snare wire 5 is subjected to a throttle manipulation, a portion of the wire gap retaining section 31 on the rear end face of the distal end chip 23 abuts against a living tissue H of a ligation target.

In the present embodiment, the two wire rods 5b, 5c at the front end of the loop section 4 of the snare wire 5 are retained to be spaced from each other by means of the wire gap retaining section 31 on the rear end face of the distal end chip 23. Thus, when the loop section 4 of the snare wire 5 is subjected to a throttle manipulation (when the living tissue H of a ligation target is ligated by means of the snare wire 5), the snare wire can be reliably abutted against the living tissue H of a ligation target in a wide area by means of a portion of this wire gap retaining section 31. Therefore, at an abutment section at the front end of the loop section 4 of the snare wire 5, the loop section 4 can be reliably prevented from cutting into the living tissue H of a ligation target.

FIG. 10 shows a third embodiment of the present invention. The present embodiment changes a configuration of the detention snare 2 of the medical ligation apparatus 1 according to the first embodiment (reefer to FIGS. 1 to 8) as follows.

That is, in a detention snare 2 according to the present embodiment, a needle shaped engaging claw 32 is protruded backwardly on the rear end face of a distal end chip 23. Further, a needle shaped engaging claw 33 is protruded forwardly on the front end face of a rear end ring 24.

In the detention snare 2 according to the present embodiment, when a loop section 4 of a snare wire 5 is subjected to a throttle manipulation, the distal end chip 23 and the rear end ring 24 can be engagingly locked with the living tissue H of a ligation target in a state in which the engaging claw 32 on the rear end face of the distal end chip 23 and the engaging claw 33 on the front end face of the rear end ring 24 are punctured into the living tissue H of a ligation target, respectively. Thus, the detention snare 2 can be prevented from slipping off and dropping from the living tissue H of a ligation target.

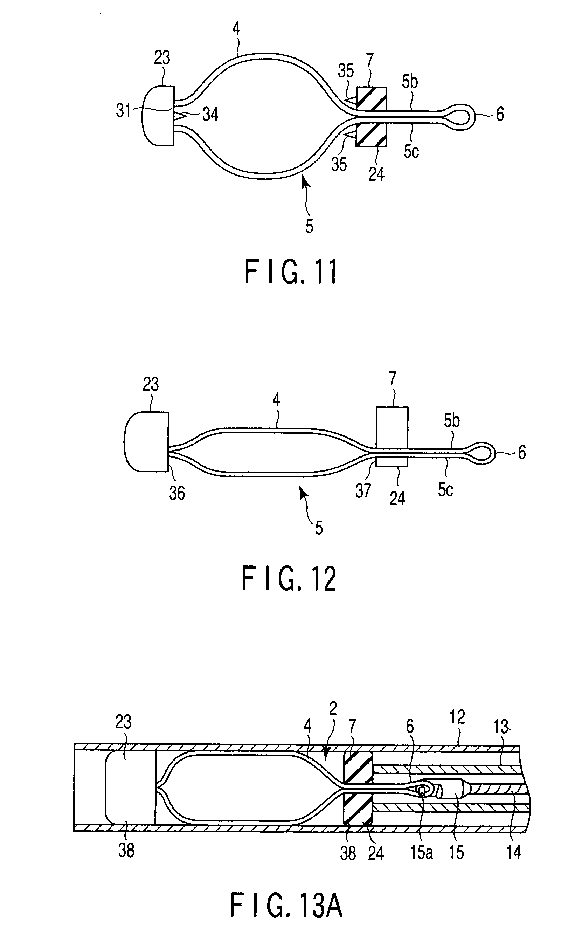

FIG. 11 shows a fourth embodiment of the present invention. The present embodiment changes a configuration of the detention snare 2 of the medical ligation apparatus 1 according to the second embodiment (refer to FIG. 9) as follows.

That is, in the present embodiment, a needle shaped engaging claw 34 is protruded backwardly at a wire gap retaining section 31 on a rear end face of a distal end chip 23. Further, a plurality of needle shaped engaging claws 35 are protruded forwardly on a front end face of a rear end ring 24.

In a detention snare 2 according to the present embodiment, when a loop section 4 of a snare wire 5 is subjected to a throttle manipulation, the distal end chip 23 and the rear end ring 24 can be engagingly locked with a living tissue H of a ligation target in a state in which the engaging claw 34 on the rear end face of the distal end chip 23 and the engaging claw 35 on the front end face of the rear end ring 24 are punctured into the living tissue H of a ligation target, respectively. Thus, according to the present embodiment as well, the detention snare 2 can be prevented from slipping off and dropping from the living tissue H of a ligation target in the same manner as the detention snare 2 according to the third embodiment.

FIG. 12 shows a fifth embodiment of the present invention. The present embodiment changes a configuration of the detention snare 2 of the medical ligation apparatus 1 according to the first embodiment (refer to FIGS. 1 to 8) as follows.

That is, in a detention snare 2 according to the present embodiment, a fixing section 36 is provided to fix a distal end of a loop section 4 of a snare wire 5 at a position deviating from a center line position on a rear end face of a distal end chip 23. Further, in a rear end ring 24, press-fit holes 37 of two wire rods 5b, 5c of the snare wire 5 are provided at positions corresponding to the fixing section 36 of the distal end chip 23.

In the detention snare 2 according to the present embodiment, the fixing section 36 of the loop section 4 of the snare wire 5 is disposed at the position deviating from the center line position on the rear end face of the distal end chip 23, and the press-fit hole 37 of the rear end ring 24 is disposed at the position corresponding to the fixing section 36 of this distal end chip 23. Thus, a resection face H1 of a living tissue H is set by means of a resection snare 25 at a position which is the furthest away from the fixing section 36 of the distal end chip 23 or the press-fit hole 37 of the rear end ring 24, whereby a margin can be increased between the resection face H1 of the living tissue H caused by the resection snare 25 and the loop section 4 of the snare wire 5 of the detention snare 2.

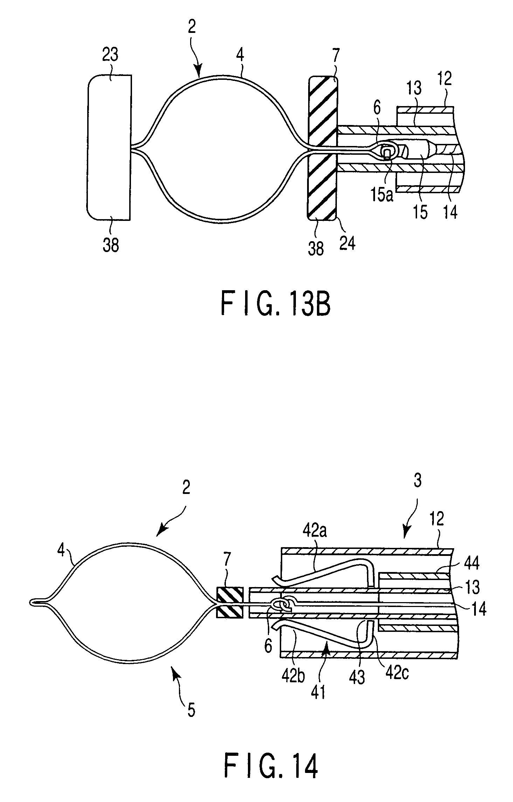

FIGS. 13A and 13B each show a sixth embodiment of the present invention. The present embodiment changes a configuration of the medical ligation apparatus 1 according to the first embodiment (refer to FIGS. 1 to 8) as follows.

That is, in a distal end chip 23 and a rear end ring 24 of a detention snare 2 are formed of an elastically deformable elastic element 38. The elastic element 38 for these distal end chip 23 and rear end ring 24 is retained in a reference shape whose diameter is greater than that of an outer sheath 12, as shown in FIG. 13B, in a natural state, for example. Further, as shown in FIG. 13A, in a state in which the detention snare 2 is housed in the outer sheath 12, the elastic element 38 for these distal end chip 23 and rear end ring 24 is housed in the outer sheath 12 while it is elastically deformed in a compression shape compressed to have the same diameter as that of the outer sheath 12.

In the detention snare 2 according to the present embodiment, as shown in FIG. 13B, when the detention snare 2 is protruded to the outside of the outer sheath 12, the elastic element 38 for the distal end chip 23 and the rear end ring 24 is elastically deformed in a state in which it is restored to a reference shape whose diameter is greater than that of the outer sheath 12. Thus, when a loop section 4 of a snare wire 5 is subjected to a throttle operation (when a living tissue H of a ligation target is ligated by means of the snare wire 5), the snare wire can be reliably abutted in a wide area with the living tissue H of a ligation target by means of the distal end chip 23 and the rear end ring 24 formed in a reference shape having this greater diameter. Thus, at the abutment sections of the front end and rear end of the loop section 4 of the snare wire 5, the loop section 4 can be reliably prevented from cutting into the living tissue H of a ligation target.

Further, as shown in FIG. 13A, in a state in which the detention snare 2 is housed in the outer sheath 12, the elastic element 38 of these distal end chip 23 and rear end ring 24 is housed in the outer sheath 12 while it is elastically deformed in the compression shape compressed to have the same diameter as that of the outer sheath 12. Therefore, the distal end chip 23 and the rear end ring 24 formed in a reference shape having a greater diameter can be prevented from being protruded to the outside of the outer sheath 12 during insertion into an endoscope channel or the like, and workability of inserting the medical ligation apparatus 1 into a body can be improved.

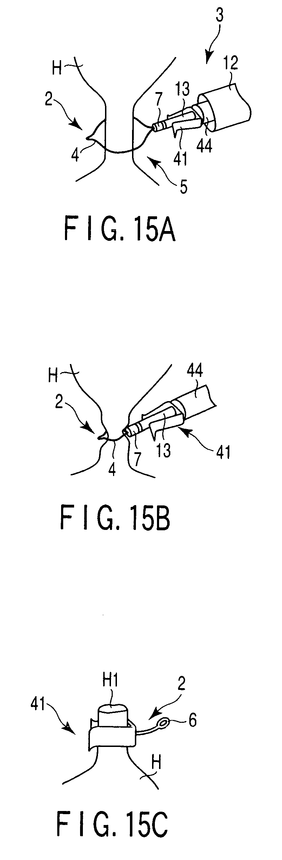

FIGS. 14 and 15A to 15C each show a seventh embodiment of the present invention. The present embodiment changes a configuration of the medical ligation apparatus 1 according to the first embodiment (refer to FIGS. 1 to 8) as follows.

That is, in the present embodiment, as shown in FIG. 15C, a clip member 41 is provided to be mounted in an externally engaged state on a detention section of a detention snare 2. As shown in FIG. 14, this clip member 41 has: a pair of pinch pieces 42a, 42b biased in a direction in which a distal end side is closed; and a coupling section 42c communicating between proximal ends of these pinch pieces 42a, 42b. A circular hole 43 into which a manipulating tube 13 is inserted is formed at an axial part of the coupling section 42c. Then, this clip member 41 is set in a state in which a distal end of the manipulating tube 13 is inserted into the circular hole 43 of the coupling section 42c.

In addition, in a snare manipulating instrument 3, a clip manipulating tube 44 is inserted to be movable in an axial direction between an outer sheath 12 and the manipulating tube 13. The clip member 41 is manipulated to be forwardly pushed out together with a manipulation for this clip manipulating tube 44 to be manipulated to be forwardly pushed out.

When using a medical ligation apparatus 1 according to the present embodiment, the detention snare 2 is inserted into a body through the inside of a channel 9 of an endoscope 8 according to procedures identical to those of the first embodiment. Then, the detention snare 2 is forwardly protruded from a distal end of the outer sheath 12 together with the manipulating tube 13 and the clip member 41. At this time, a loop section 4 of the detention snare 2 is expanded in a loop shape, as shown in FIG. 14, by means of elasticity of a snare wire 5 itself.

Then, as shown in FIG. 15A, the snare manipulating instrument 3 is moved in a state in which a target living tissue H is inserted into the loop section 4 of the detention snare 2. In this state, a throttle member 7 is pushed out forwardly (toward the distal end side of the loop section 4) by means of the manipulating tube 13. In this manner, the loop section 4 of the snare wire 5 is throttled. As a result, as shown in FIG. 15B, a root portion of the target living tissue H inserted into the loop section 4 is throttled by means of the loop section 4 of the snare wire 5.

Then, the clip manipulating tube 44 is manipulated to be forwardly pushed out. The clip member 41 is manipulated to be forwardly pushed out together with the manipulation of this clip manipulating tube 44. In this manner, as shown in FIG. 15C, the clip member 41 is mounted in an externally engaged state on a ligated site of the living tissue H ligated by throttling the loop section 4 of the detention snare 2 by means of the throttle member 7.

In the medical ligation apparatus 1 according to the present embodiment, a resection snare 25 is positioned by means of the clip member 41 mounted in an externally engaged state on the ligated site of the living tissue ligated by means of the detention snare 2. Thus, a sufficient margin can be provided between a resection face H1 of the living tissue H caused by the resection snare 25 and the loop section 4 of the snare wire 5.

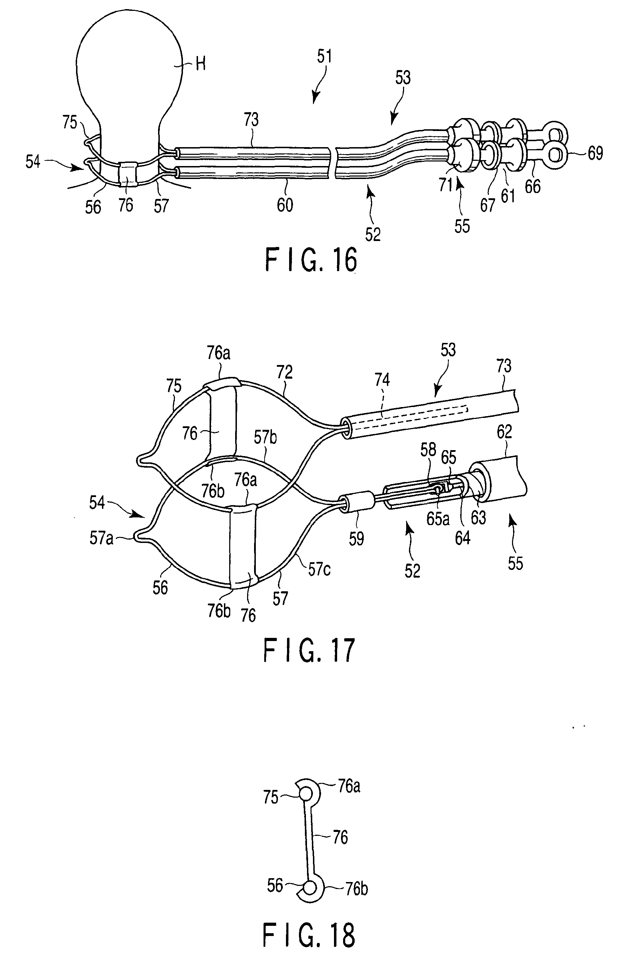

FIGS. 16 to 18 each shows an eighth embodiment of the present invention. FIG. 16 shows a general configuration of a whole medical ligation apparatus 51 according to the present embodiment. A ligating instrument 52 for ligation of a living tissue H and a resection tool 53 are provided at this medical ligation apparatus 51. The ligation instrument 52 has a detention snare 54 and a snare manipulating instrument 55 for manipulating this detention snare 54.

FIG. 17 shows the detention snare 54 according to the present embodiment. This detention snare 54 has a snare wire 57, a coupling ring 58, and a ring shaped throttle member 59. In the snare wire 57, a ligation loop section 56 is formed to be expandable in a loop shape at a distal end side. The coupling ring 58 is provided at a proximal end side of the snare wire 57. The throttle member 59 is inserted in a state in which the proximal end side of the snare wire 57 is press-fitted. The snare wire 57 is formed of a synthetic resin material having a good biocompatibility, for example.

In the detention snare 54, for example, the snare wire 57 is bent in a loop shape, and the loop section 56 is formed. Then, in the loop section 56, a bent section 57a of the snare wire 57 is disposed at a distal end side. Further, two wire rods 57b, 57c formed by bending the snare wire 57 are extended to the proximal end of the loop section 56. The two wire rods 57b, 57c extended to the proximal end of the loop section 56 are press-fitted into the throttle member 59 in a bundled state. Further, a coupling ring 58 is disposed at a portion of an extension end of the two wire rods 57b, 57c extended backwardly of the throttle member 59.

In addition, the snare manipulating instrument 55 has: an elongated insert section 60 and a frontal manipulating section 61 coupled with a proximal end of this insert section 60, as shown in FIG. 16. As shown in FIG. 17, the insert section 60 has an outer sheath 62, a manipulating tube 63, and a manipulating wire 64. The manipulating tube 63 is inserted to be movable in an axial direction inside of the outer sheath 62. Further, the manipulating wire 64 is inserted to be movable in an axial direction inside of the manipulating tube 63.

A proximal end of a hook member (snare coupling section) 65 is connected and fixed to a distal end of the manipulating wire 64. A claw shaped hook 65a is provided at a distal end of this hook member 65. The hook 65a is protruded toward a direction substantially orthogonal to the axial direction of the manipulating wire 64. The coupling ring 58 of the detention snare 54 is removably engaged with this hook 65a.

In addition, as shown in FIG. 16, the manipulating section 61 has a manipulating section main body 66 and a slider section 67 as in the first embodiment. The manipulating section main body 66 is extended toward the axial direction of the insert section 60. The slider section 67 is slidable in the axial direction of the insert section 60 along the manipulating section main body 66. Then, the distal end of the manipulating section main body 66 is connected and fixed to the proximal end of the manipulating tube 63. Further, a finger hook ring 69 is assembled to be rotatable in a axial turning direction at the proximal end of the manipulating section main body 66. In addition, the manipulating wire 64 is manipulated to be pushed and pulled together with the slide manipulation of the slider section 67.

In addition, an outer sheath grip section 71 is coupled with the proximal end of the outer sheath 62. This outer sheath 71 is retractably assembled in the axial direction of the insert section 60 at the distal end of the manipulating section main body 66. Then, the snare manipulating instrument 55 is set in a state in which the hook 65a of the hook member 65 is inserted into the coupling ring 58 of the detention snare 54 and is removably engaged therewith. In this state, this manipulating instrument is used for manipulation of the detention snare 54.

In addition, the resection instrument 53 has a resection snare 72. This resection snare 72 has: a resection snare introducing tube 73; an elongated manipulating wire 74; and a resection loop section 75.

The manipulating wire 74 is inserted to be movable in the axial direction in the tube 73. The loop section 75 is arranged at a distal end of the manipulating wire 74. The loop section 75 is designed to expand in a loop shape.

In addition, a plurality of coupling plates 76 (two coupling plates 76 in the present embodiment) are arranged between the resection loop section 75 of the resection snare 72 and the loop section 56 of the detention snare 54. As shown in FIG. 18, a substantially C-shaped upper end engagement section 76a is formed at the upper end of each coupling plate 76, and a substantially C-shaped lower end engagement section 76b is formed at the lower end thereof, respectively. Then, the upper end engagement section 76a of each coupling plate 76 is removably engaged with the resection loop section 75 of the resection snare 72 and the lower end engagement section 76b of each coupling plate 76 is removably engaged with the loop section 56 of the detention snare 54.

When using the medical ligation apparatus 51 according to the present embodiment, first, a living tissue H is ligated by means of the loop section 56 of the detention snare 54 of the ligation instrument 52. Then, the upper section of the ligated site by the loop section 56 of this detention snare 54 is resected by means of the loop section 75 of the resection snare 72 of the resection instrument 53. At the time of this work, a gap between the ligated site of the living tissue H ligated by the loop section 56 of the detention snare 54 and the resected face caused by the loop section 75 of the resection snare 72 can be allocated by means of the coupling plate 76.

In the present embodiment, two coupling plates 76 are arranged between the resection loop section 75 of the resection snare 72 and the loop section 56 of the detention snare 54. Thus, a sufficient margin can be provided between the ligated site of the living tissue H ligated by the loop section 56 of the detention snare 54 and the resected face caused by the loop section 75 of the resection snare 72. As a result, the resection snare 72 can be reliably prevented from slipping off from the upper section to the root side of the ligated site of the living tissue H.

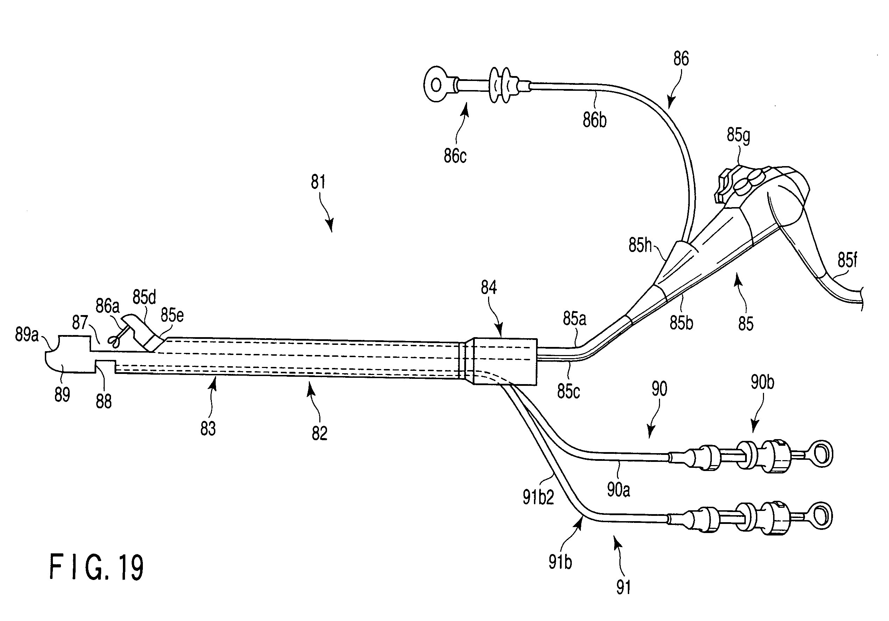

FIGS. 19 to 24 each show a ninth embodiment of the present invention. In the present embodiment, there is provided a surgical instrument system 81 for fully resecting a colon. As shown in FIG. 19, this surgical instrument system 81 has a surgery insert assisting instrument 82, an endoscope 85, and a plurality of surgical instruments (a grip forceps 86, a resection snare 90, and a ligation snare 91).

The surgery insert assisting instrument 82 has an elongated tubular insert section 83 inserted into a colon and a frontal grip section 84. The insert section 83 is formed of a resin based material consisting of a thermally plastic elastomer having flexibility. Elongated rectangular surgery side holes 88 are provided in a long axis direction of the insert section 83 on a distal end outside face of the insert section 83. Similarly, at a distal end side of the insert section 83, there is a work space 87 configured by partially cutting out an outside top of the insert section 83 relative to the surgery side hole 88.

Further, at the distal end of the insert section 83, an endoscope guide section 89 is provided at the front face of the surgery side hole 88 and the work space 87. An endoscope insert port 89a is formed at this endoscope guide section 89. The endoscope insert port 89a inserts the insert section of the colon endoscope, although not shown, used as a guide for inserting the surgery insert assisting instrument 82 into a depth of the colon. Then, the surgery insert assisting instrument 82 is inserted into the depth of the colon in a state in which the surgery insert assisting instrument 82 is externally inserted into the insert section of the colon endoscope.

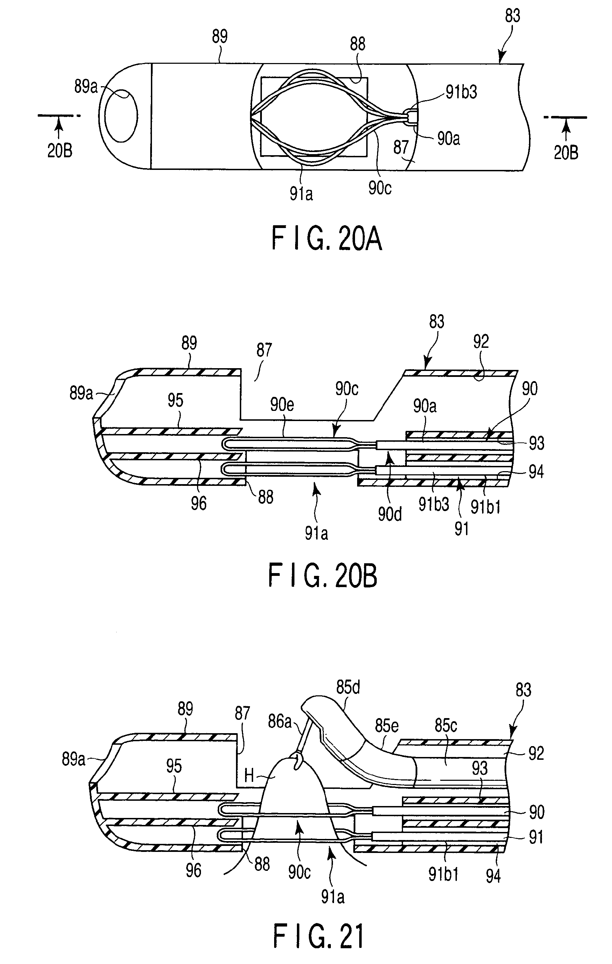

In addition, as shown in FIG. 20B, at the insert section 83 of the surgery insert assisting instrument 82, an endoscope insert tube channel 92, a resection snare insert tube channel 93, and a ligation snare insert tube channel 94 are extended, respectively, along the axial direction of the insert section 83. The endoscope insert tube channel 92 is formed to communicate from the frontal grip section 84 to the work space 87. The resection snare insert tube channel 93 and the ligation snare insert tube channel 94 are formed to communicate from the frontal grip section 84 to the vicinity of the rear end position of the surgery side hole 88.

The endoscope 85 has: an elongated insert section 85a inserted into a body; and a frontal manipulating section 85b coupled with a proximal end of this insert section 85a. The insert section 85a has: an elongated flexible tube section 85c having flexibility; a hard distal end 85d disposed at the most distal position; and a curve section 85e which can be deformed in a curved shape. At the inside of the insert section 85a, there are provided: a plurality of manipulating wires, a control signal cable, a light guide cable, a water supply or intake tube and a variety of probe channels or the like.

The endoscope 85 according to the present embodiment is provided as a side viewing endoscope in which a viewing direction of an observation optical system of the distal end 85d is oriented in a direction which is orthogonal from the axial direction of the insert section 85a or which is oriented substantially backwardly. On a side face of the distal end 85d, there are provided: an illumination window, an observation window; a channel opening section for air supply and water supply; and a forceps channel opening section or the like compatible with an intake port. An emission end of the light guide cable is disposed to be opposed to another on the internal face of the observation window. An objective lens of the observation optical system and an electronic image pickup element (CCD) or the like for picking up an image of an object observed by this objective lens are arranged on the internal face of the observation window. Then, illumination light is projected from the emission end of a distal end of the light guide cable through the illumination window so that an object illuminated by the projected illumination light is picked up as an image by means of an electronic image pickup element.

At the distal end 85d of the endoscope 85, a forceps rise base, although not shown, is provided in an opening section for a forceps channel. This forceps rise base is manipulated by a forceps rise knob, although not shown, which is provided at the manipulating section 85b. Then, a protrusion direction of a distal end grip section 86a of a grip forceps 86 described later inserted into the forceps channel is deflected by means of a rise manipulation of this forceps rise base.

In addition, a proximal end of a universal cord 85f is coupled with the manipulating section 85b. The other end of this universal cord 85f is connected to a light source instrument via a connector, although not shown. Then, the illumination light from the light source instrument is supplied to a light guide cable in the universal cord 85f. An electrical cable is further connected to the connector. This electrical cable is connected to a video processor via an electrical connector. Then, an endoscope image of an object imaged by the observation optical system is converted into an electrical signal by means of an electronic image pickup element. Then, the electrical signal is transmitted to the video processor via the control signal cable, and is displayed on a display monitor, although not shown.

Further, at the manipulating section 85b, there are provided: a manipulating knob 85g for curve operation; a forceps channel insert port 85h; and a forceps rise knob or the like, although not shown. Then, a manipulating wire in the insert section 85a is manipulated to be pulled by means of manipulation of the manipulating knob 85g and the curve section 85e is manipulated to be curved vertically or horizontally for changing an orientation of the distal end 85d.

In addition, the grip forceps 86 is inserted into a forceps channel insert port 85h. This grip forceps 86 has an elongated insert section 86b, a frontal manipulating section 86c, and a distal end grip section 86a. The manipulating section 86c is coupled with a proximal end of the insert section 86b. The distal end grip section 86a is disposed at a distal end of the insert section 86b.

The grip forceps 86 is inserted into the surgical instrument insert channel through the forceps channel insert port 85h of the endoscope 85, and is inserted into the distal end side of the endoscope 85. Further, the distal end grip section 86a of the grip forceps 86 is protruded from the distal end 85d of the insert section 85a into a body cavity. At this time, by manipulating a forceps rise knob (not shown) of the manipulating section 85b, the forceps rise base of the forceps channel opening section of the distal end 85d is manipulated to rise to deflect the protrusion direction of the distal end grip section 86a inserted into the forceps channel.

In addition, the resection snare 90 has a tubular insert section 90a. A frontal manipulating section 90b is provided at the front face of this insert section 90a. This frontal manipulating section 90b is connected to a high frequency power supply instrument, although not shown. The loop section 90c formed of a metallic loop wire 90e is disposed at a distal end of the insert section 90a. A metallic manipulating wire 90d is inserted retractably in an axial direction of the insert section 90a. A loop section 90c is coupled with a distal end of this manipulating wire 90d. Then, the manipulating wire 90d is retractably driven in an axial direction by means of the frontal manipulating section 90b. At this time, the distal end loop section 90c is manipulated to be protruded and recessed from the inside of the insert section 90a via the manipulating wire 90d.

The ligation snare 91 is configured in the same way as the medical ligation apparatus 1 according to the first embodiment (refer to FIGS. 1 to 8). This ligation snare 91 has: a detention snare 91a shown in FIG. 20A, which is a ligation instrument; and a snare manipulating instrument 91b for manipulating this detention snare 91a.

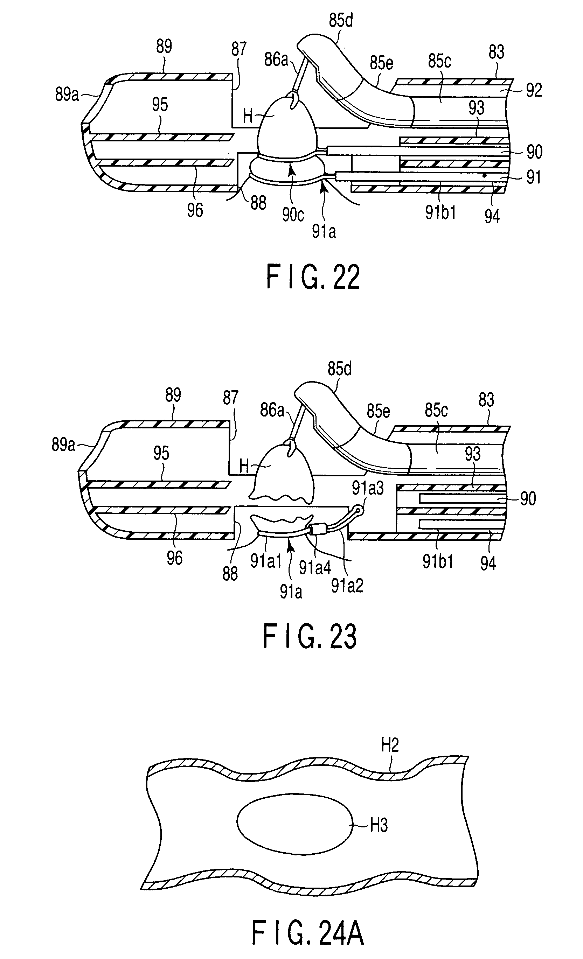

As shown in FIG. 23, the detention snare 91a has: a snare wire 91a2 having formed thereat a loop section 91a1 for ligation which is expandable in a loop shape at its distal end side; a coupling ring 91a3 provided at the proximal end side of this snare wire 91a2; and ring shaped throttle member 91a4 into which the proximal end side of the snare wire 91a2 is inserted in a press-fit state.

The snare manipulating instrument 91b has a tubular insert section 91b1 and a frontal manipulating section 91b2 arranged at the front face of this insert section 91b1. The insert section 91b1 has an outer sheath 91b3, a manipulating tube, although not shown, inserted to be movable in an axial direction in the inside of the sheath; and a manipulating wire, although not shown, inserted to be movable in an axial direction in the inside of the tube.

A proximal end of a hook member, although not shown, is connected and fixed to a distal end of the manipulating wire. The coupling ring 91a3 of the detention snare 91a is removably engaged at a distal end of this hook member.

When the surgical instrument system 81 is used, the insert section 85a of the endoscope 85 is inserted into the endoscope insert tube channel 92 from the frontal grip section 84 of the surgery insert assisting instrument 82, and is introduced into the work space 87. The resection snare 90 is inserted into the resection snare insert tube channel 93 from the frontal grip section 84, and is introduced into the work space 87 in the same way. Similarly, the ligation snare 91 is also inserted into the ligation snare insert tube channel 94 from the frontal grip section 84, and is introduced into the work space 87.

In addition, as shown in FIG. 20B, in the endoscope guide section 89 of the surgery insert assisting instrument 82, at a distal end side of the surgery side hole 88, a substantially planar resection snare retainer 95 and a ligation snare retainer 96 are formed in substantially parallel to the surgery side hole 88, respectively. These resection snare retainer 95 and the ligation snare retainer 96 are arranged at an interval substantially equal to each channel diameter at a position relative to the resection snare channel 93 and the ligation snare insert tube channel 94.

Then, as shown in FIG. 20A, the loop section 90c of the resection snare 90 and the loop section 91a1 of the detention snare 91a are disposed to surround the periphery of the surgery side hole 88 of the surgery insert assisting instrument 82. At this time, a distal end of the loop section 90c of the resection snare 90 is set in a state in which it abuts against a lower face side of the resection snare retainer 95 of the endoscope guide section 89, and a distal end of the loop section 91a1 of the detention snare 91a is set in a state in which it abuts against a lower face side of the ligation snare retainer 96, respectively. In this manner, a living tissue H pulled up from the surgery side hole 88 abuts against the loop section 90c of the resection snare 90 and the loop section 91a1 of the detention snare 91a, whereby the loop sections 90c and 91a1 are prevented from being unnecessarily lifted.

Now, a function of the present embodiment having the above configuration will be described here. When the surgical instrument system 81 according to the present embodiment is used, first, the surgery insert assisting instrument 82 is externally inserted into a direct viewing type colon endoscope, and then, the colon endoscope is inserted into a site targeted for colon surgery. Then, the surgery insert assisting instrument 82 is inserted into a surgery target site along this colon endoscope. Then, the colon endoscope is removed from the surgery insert assisting instrument 82.

After removing the colon endoscope, the endoscope 85 is inserted into the surgery insert assisting instrument 82. A distal end 85d of the insert section 85a of the endoscope 85 is inserted into a diseased site targeted for colon surgery. In this way, the endoscope 85 is inserted into the surgery insert assisting instrument 82, and the surgery side hole 88 of the surgery insert assisting instrument 82 is aligned with the surgery target site in the observation field of view using the endoscope 85. In this state, as shown in FIG. 20A, the loop section 90c of the resection snare 90 and the loop section 91a1 of the detention snare 91a are disposed to surround the periphery of the surgery side hole 88 of the surgery insert assisting instrument 82. At this time, the loop section 90c of the resection snare 90 and the loop section 91a1 of the detention snare 91a are set as follows, respectively. That is, the loop section 90c of the resection snare 90 is set so that its distal end abuts against a lower face side of the resection snare retainer 95 of the endoscope guide section 89. The loop section 91a1 of the detention snare 91a is set so that its distal end abuts against the lower face side of the ligation snare retainer 96.

Then, the grip forceps 86 is inserted into the forceps channel insert port 85h of the endoscope 85. The distal end grip section 86a of the grip forceps 86 inserted into the forceps channel of the endoscope 85 is protruded to the outside from a forceps channel opening section of the distal end 85d of the endoscope 85. In this state, the distal end grip section 86a of the grip forceps 86 is moved toward the surgery side hole 88.

Then, a surgery target site of a colon is gripped by the distal end grip section 86a of the grip forceps 86. In this state, the curve section 85e of the endoscope 85 is curved, whereby, as shown in FIG. 21, the distal end 85d of the insert section 85a is manipulated to rise, and the surgery target site of the colon is manipulated to be pulled up to the work space section 87 through the surgery side hole 88. At this time, the living tissue H of the surgery target site of the colon manipulated to be pulled up by the distal end grip section 86a of the forceps 86 is pulled up to the work space 87 through the preset loop section 91a1 and the loop section 90c.

When this living tissue H is pulled up, the distal end of the loop section 91a1 is abutted against the ligation snare retainer 96, and the distal end of the loop section 90c is abutted against the resection snare retainer 95, respectively. Thus, the living tissue H pulled up through the surgery side hole 88 abuts against the loop section 90c of the resection snare 90 and the loop section 91a1 of the detention snare 91a, whereby the loop sections 90c, 91a1 are prevented from being unnecessarily lifted. In this manner, a gap between the loop section 90c of the resection snare 90 and the loop section 91a1 of the detention snare 91a is retained at a proper length.

Then, the loop section 90c of the resection snare 90 and the loop section 91a1 of the detention snare 91a are throttled by manipulating the frontal manipulating section 90b of the resection snare 90 and the snare manipulating instrument 91b of the ligation snare 91. In this manner, the proper positions of the living tissue H can be throttled, respectively. In this state, the living tissue H is cut by supplying high frequency power to the loop wire 90e of the loop section 90c via the frontal manipulating section 90b of the resection snare 90. Then, the detention snare 91a is isolated from the snare manipulating instrument 91b, and the living tissue H is ligated in a state in which the loop section 91a1 of the detention snare 91a is fixed to be cut into the living tissue H by manipulating the snare manipulating instrument 91b of the ligation snare 91.

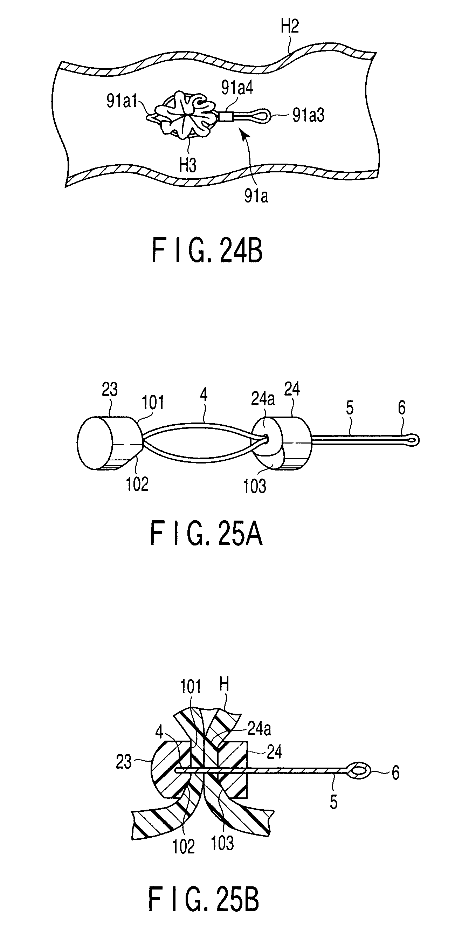

At this time, in the present embodiment, the surgery side hole 88 is formed in the shape of an elongated longitudinally rectangular opening section in an axial direction of the insert section 83. Thus, the sectional shape of the living tissue H targeted for surgery pulled up from the surgery side hole 88 can also be shaped into an elongated longitudinally rectangular prism shaped opening section close to the shape of the opening section of this surgery side hole 88, as shown in FIG. 24A. Thus, the shape of a resection hole H3 formed at the time of fully resecting a colon H2 can be reduced in length in a direction orthogonal to an axial direction, as compared with a length in the axial direction of the colon H2.

The following advantageous effect is attained with the above configuration. That is, in the surgery insert assisting instrument 82 according to the present embodiment, the resection snare insert tube channel 93 of the insert section 83 of the surgery insert assisting instrument 82 is disposed at an upper position of the ligation snare insert tube channel 94, in FIG. 20B. In this manner, a sufficient margin can be provided between a ligated site of the living tissue H ligated by the loop section 91a1 of the detention snare 91 and a resection face caused by the loop section 90c of the resection snare 90. As a result, when the resection snare 90 throttles and resects an upper section of the ligated site of the living tissue H, the resection snare can be reliably prevented from slipping off from the upper portion to the root side.

In addition, in the present embodiment, the surgery side hole 88 is formed in the shape of an elongated longitudinally rectangular prism shaped opening section in the axial direction of the insert section 83. Thus, when a colon is fully resected, a living tissue targeted for surgery is manipulated to be pulled up to the work space 87 through the inside of the opening section of the surgery side hole 88, and a root portion of the pulled up living tissue targeted for surgery can be ligated and sutured to be efficiently folded by one ligating instrument, i.e., the detention snare 91.

The resection snare 90 and the ligation snare 91 shown in the present embodiment may be configured as shown in the first to eighth embodiments of the present invention without being limited to that of the present embodiment.

FIGS. 25A and 25B each show a tenth embodiment of the present invention. The present embodiment changes a configuration of the detention snare 2 according to the first embodiment (refer to FIGS. 1 to 8) as follows.

That is, in a detention snare 2 according to the present embodiment, as shown in FIG. 25A, a tapered section 102 is provided to be expandable in an obliquely front direction at the lower side of an insert section of a snare wire 5 on a rear end face 101 of a distal end chip 23. Similarly, at a distal end contact face 24a of a rear end ring 24 as well, a tapered section 103 is provided to be expandable in an obliquely rear direction at the lower side of an insert section of the snare wire 5. When a living tissue H is throttled by means of the detention snare 2, as shown in FIG. 25B, the tapered section 102 of the distal end chip 23 and the tapered section 103 of the rear end ring 24 are disposed at a root section of the living tissue H sandwiched between the rear end face 101 of the distal end chip 23 and the distal end contact face 24a of the rear end ring 24.

The following advantageous effect is attained with the above configuration. That is, as in the detention snare 2 according to the present embodiment, the tapered section 102 is provided on the rear end face 101 of the distal end chip 23, and similarly, the tapered section 103 is provided on the distal end contact face 24a of the rear end ring 24, whereby, as shown in FIG. 25B, the throttling of part (root side) of the living tissue H sandwiched between the rear end face 101 of the distal end chip 23 of the detention snare 2 and the distal end contact face 24a of the rear end ring 24 can be loosened.

In the case where the tapered section 103 and the tapered section 104 are not present, a ligation force of the living tissue H caused by the detention snare 102 is determined depending on only a throttling force between the rear end face 101 of the distal end chip 23 of the detention snare 102 and the distal end contact face 24a of the rear end ring 24. Thus, in the case where the throttling force between the rear end face 101 of the distal end chip 23 and the distal end contact face 24a of the rear end ring 24 is large, and the ligated portion of the living tissue H is excessively tightened (in the case where the ligating force quantity is high), there is a possibility that crush of the living tissue H or necrosis due to shortage of blood flow occurs. Conversely, in the case where the ligation force quantity is low, efficient ligation cannot be carried out. In contrast, as in the present embodiment, in the case where the tapered section 103 is provided at the rear end face 101 of the distal end chip 23 and the tapered section 104 is provided at the distal end contact face 24a of the rear end ring 24, respectively, the ligation force quantity continuously changes at a site sandwiched between the tapered sections 103 and 104. Thus, in any site of this range, membrane faces of the living tissue H can be abutted against each other with a proper force quantity, and thus, the living tissue can be reliably ligated without excessively tightening the living tissue H.

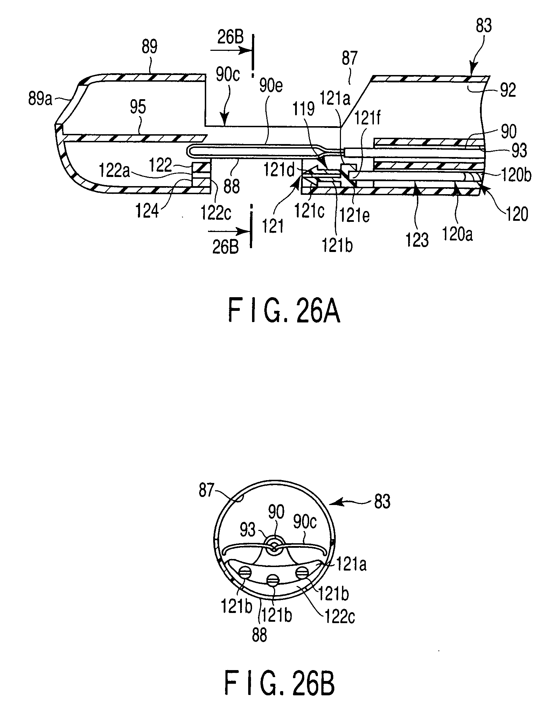

FIGS. 26A to 26D each show an eleventh embodiment of the present invention. The present embodiment is provided as a modified example of the surgical instrument system 81 for full colon resection shown in the ninth embodiment (refer to FIGS. 19 to 24).

That is, although the ninth embodiment has shown a case in which the retention snare 91a is used as a ligation instrument for a living tissue H, a ligation instrument 119 and its manipulating instrument 120 shown in FIG. 26A are provided instead of the retention snare in the present embodiment. The other constituent elements are identical to those according to the ninth embodiment. Thus, the same constituent elements as those according to the ninth embodiment are designated by the same reference numerals, a duplicate description is omitted here.

The ligation instrument 119 has a ligation instrument main body 121 and a receptacle plate 122. The ligation instrument main body 121 has a sublunate planar member 121a made of a hard resin, as shown in FIG. 26B. This planer member 121a is set in a shape such that it is inscribed at an insert section 83 of a surgery insert assisting instrument 82.

A plurality of puncture needles 121b (three puncture needles in the present embodiment) are protruded at the front face of this planer member 121a. These three puncture needles 121b are disposed along an arc shaped internal face of the insert section 83. A return section 121c whose diameter is greater than that of an axial section is formed at a distal end of each puncture needle 121b. A distal end face of this return section 121c is formed in a tapered conical shape. Further, at a distal end face of each puncture needle 121b, a slot 121d is formed along its axial direction.

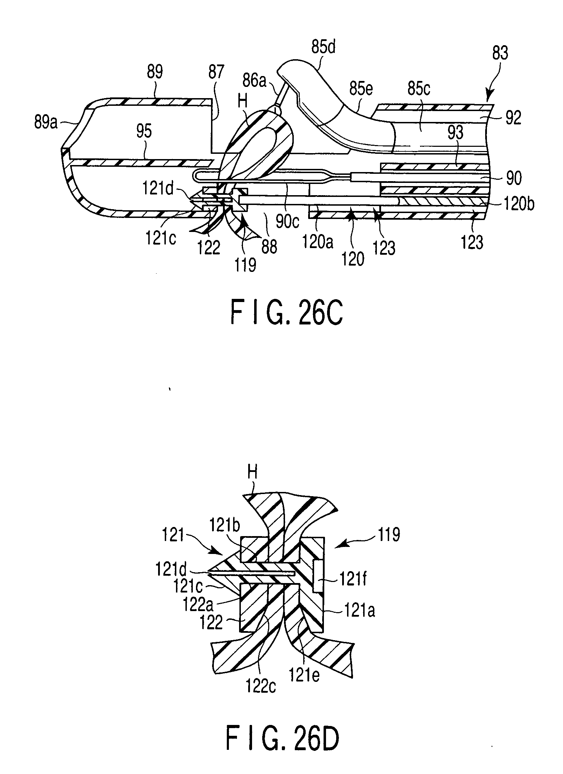

In addition, as shown in FIG. 26D, a tapered section 121e expendable in an obliquely rear orientation at the lower side of each puncture needle 121b is provided at the front face of the planer member 121a. Further, at the rear face of the planer member 121a, an engagement hole 121f is provided at a position corresponding to each puncture needle 121b. One engagement hole 121f may be provided at the center position on the rear face of the planer member 121a.

In addition, the receptacle plate 122 of the ligation instrument 119 is formed of a planer member which is formed in the substantially same shape as the planer member 121a of the ligation instrument main body 121. On this receptacle plate 122, a through hole 122a is formed at a position corresponding to each puncture needle 121b of the planer member 121a. Further, a tapered section 122c expandable in an obliquely front direction at the lower side of each through hole 122a is provided on the rear face of the receptacle plate 122.

In addition, at the insert section 83 of the surgery insert assisting instrument 82 according to the present embodiment, a ligation tool push-out member insert tube channel 123 is arranged backwardly of a surgery side hole 88. This ligation instrument push-out member insert tube channel 123 is formed to communicate from a frontal grip section 84 to the vicinity of the rear end position of the surgery side hole 88 as in the ligation snare insert tube channel 94 according to the ninth embodiment.

The ligation instrument manipulating instrument 120 has a substantially hard bar shaped member 120a and a wire member 120b. The bar shaped member 120a has a length from the distal end side of the ligation instrument push-out insert tube channel 123 to the distal end side of the surgery side hole 88. Then, a distal end of this bar shaped member 120a is removably engaged with the engagement hole 121f of the ligation instrument main body 121.

In addition, the wire member 120b is retractably inserted in an axial direction into the ligation instrument push-out member tube channel 123. A distal end of this wire member 120b is connected to a rear end of the bar shaped member 120a. Further, a rear end of the wire member 120b is extended up to the frontal grip section 84 of the surgery insert assisting instrument 82. Then, the wire member 120b is retractably driven in an axial direction in the ligation instrument push-out member insert tube channel 123 by means of manipulation of the frontal grip section 84. At this time, the ligation instrument manipulating instrument 120 is slidable forwardly and backwardly by a length at which the distal end of the bar shaped member 120a moves from the rear end side to the distal end side of the surgery side hole 88.

Then, in the case where the ligation instrument 119 is assembled at the insert section 83 of the surgery insert assisting instrument 82 according to the present embodiment, as shown in FIG. 26A, the ligation instrument main body 121 of the ligation instrument 119 is set to be disposed between a frontal end of the surgery side hole 88 and the ligation instrument push-out member insert tube channel 123. In addition, the receptacle 122 of the ligation instrument 119 is set to be disposed at a position corresponding to the ligation instrument main body 121 at the distal end side of the surgery side hole 88. An engagement groove 124 in which the receptacle plate 122 is engaged with the distal end side of the surgery side hole 88 is provided at the insert section 83 of the surgery insert assisting instrument 82 according to the present embodiment.

Further, a resection snare 90 protrudes from a resection snare insert tube channel 93 as in the ninth embodiment and is set in a state in which a loop section 90c abuts against a lower face of a snare retainer 95.

Now a function of the present embodiment having the above configuration will be described here. When a surgical instrument system 81 according to the present embodiment is used, the surgery insert assisting instrument 82 is inserted into a surgery target site of colon in accordance with procedures similar to those according to the ninth embodiment. Then, in accordance with the procedures similar to those according to the ninth embodiment, a living tissue H is inserted into the surgery side hole 88 and the loop section 90c of the resection snare 90, and then, is pulled up into a work space 87 while the tissue is gripped by a distal end grip section 86a of a grip forceps 86.

In this state, the ligation instrument manipulating instrument 120 is protruded forwardly by means of manipulation at a frontal side, although not shown. At the time of this manipulation, the planar member 121a of the ligation instrument main body 121 is manipulated to be forwardly pushed out. Thus, as shown in FIG. 26C, the three puncture needles 121b of the ligation instrument main body 121 are punctured into a root portion of the living tissue H pulled up into the work space 87, and further, these needles are engaged with the through holes 122a of the receptacle plate 122 while they are slackened by the slot 121d. At this time, when the planer member 121a of the ligation instrument main body 121 is punctured into the living tissue H, the bar shaped member 120a of the ligation instrument manipulating instrument 120 functions as a guide so that the puncture needles 121b can be reliably engaged with the through holes 122a of the receptacle plate 122, respectively. At a time point at which the ligation instrument main body 121 has been moved to a frontal terminal position, the return section 121c is fixed in abutment against a distal end side of the receptacle plate 122.

In this state, the living tissue H is cut by throttling the loop section 90c of the resection snare 90, and then, supplying a high frequency. FIG. 26D shows a diseased part after the living tissue H has been resected. Here, the living tissue H is firmly ligated between the planer member 121a of the ligation instrument main body 121 and the receptacle plate 122.

The following advantageous effect is attained with the above-described configuration. That is, in the surgery insert assisting instrument 82 according to the present embodiment, the tapered section 122c is provided on the receptacle plate 122 and the tapered section 121e is provided at the planer member 121a of the ligation instrument main body 121, respectively, downwardly of a puncture point caused by the puncture needle 121b of the ligation instrument main body 121. In this manner, the tightening of the living tissue H sandwiched between the planer member 121a of the ligation instrument main body 121 and the receptacle plate 122 is loosened.

Here, although it is necessary to strongly tighten the living tissue H in order to reliably close the living tissue H, if the tightening is excessive, there is a possibility that crush of the living tissue H or necrosis due to shortage of a blood flow occurs. As shown in the present embodiment, the tightening of the living tissue H is continuously loosened by means of the tapered section 122c of the receptacle plate 122 and the tapered section 121e of the planer member 121a of the ligation instrument main body 121, whereby, in this loosed range, membrane faces of the living tissue H can be brought into pressure contact with each other by a reliably proper tightening force quantity, and safe and reliable tissue adhesion can be achieved.

Further, the present invention is not limited to the above-described embodiments. Of course, various modifications can occur without deviating from the spirit of the invention.

Now, other characterizing technical features of the present application will be additionally described as follows.

### Description

### (Additional feature 1)

A medical ligation apparatus comprising:
a detention snare comprising a snare wire having formed thereat a loop section for ligation which is expandable in a loop shape at a distal end side thereof, a coupling ring provided at a proximal end side of the snare wire, and a ring shaped throttle member into which the proximal end side of the snare wire is inserted in a press-fitted state; and
a snare manipulating instrument having provided thereat a snare coupling section at which the coupling ring is removably coupled with a distal end of an elongated insert section to be inserted into a body,
the medical ligation apparatus ligating a ligation target in a loop section of the snare wire to manipulate the loop section of the snare wire to be throttled in a throttle direction by manipulating the throttle member to be pushed out to a distal end side of the snare wire in a state in which the detention snare is coupled with the snare coupling section and the ligation target is inserted into the loop section of the snare wire,
wherein there is provided living tissue cut-in preventing means of the ligation target which is disposed at least at a rear end side of the loop section of the snare wire when the living tissue of the ligation target is ligated by means of the snare wire, the means comprising a contact face on which a contact area with the living tissue of the ligation target is greater than a sectional area of the snare wire.

### (Additional feature 2)

The medical ligation apparatus according to additional feature 1, wherein the living tissue cut-in preventing means has slip preventing means with a living tissue of a ligation target on the contact face.

### (Additional feature 3)

The medical ligation apparatus according to additional feature 1 or additional feature 2, wherein the living tissue cut-in preventing means are provided forwardly and backwardly of a loop section of the snare wire, respectively.

### (Additional feature 4)

The medical ligation apparatus according to additional feature 1, wherein the living tissue cut-in preventing means has a cutaway setting section for, when resecting an upper side of a ligated site at a root side of a living tissue of a ligation target ligated by the detention snare, setting a predetermined cutaway between a resection face at the upper side of the ligated site and the ligated site.

### (Additional feature 5)

The medical ligation apparatus according to additional feature 4, wherein the cutaway setting section is formed of a clip member independent of the throttle member.

### (Additional feature 6)

A surgical instrument comprising:
a surgery insert assisting instrument comprising:
   an opening section having formed at an insert section inserted into a cavity in a body at least an endoscope insert channel, a ligation channel into which a ligation surgical instrument for inserting a ligation instrument for ligation of a living tissue is inserted, and a resection channel into which a resection surgical instrument for resecting a ligated site of the living tissue ligated by the ligation instrument is inserted, the opening section being disposed at a distal end of the insert section to insert the living tissue of the ligation target into one side of a distal end outer periphery face of the insert section; and a work space section formed at the other end of the distal end outer periphery face to manipulate the living tissue of the ligation target to be pulled up from the opening section;
   the ligation surgical instrument inserted into the ligation channel and set to be disposed at a position surrounding the periphery of the opening section;
   an endoscope inserted into the work space section through the endoscope insert channel;
   a grip surgical instrument having provided thereat a grip section which grips the living tissue at a distal end of an insert section inserted into the work space section through the endoscope insert channel;
   tissue pulling up means for manipulating the living tissue of the ligation target to be pulled up through an inside of the opening section by means of a curve manipulation of the endoscope in a state in which the living tissue is gripped by means of the grip section of the grip surgical instrument;
   ligation means for ligating by means of the ligation surgical instrument the living tissue of the ligation target pulled up by the tissue pulling up means; and
   a resection surgical instrument inserted into the resection channel, the resection surgical instrument resecting the ligated site of the living tissue ligated by the ligation means,
wherein a shape of the opening section of the surgery insert assisting instrument is set so as to reduce a length in a direction orthogonal to the axial direction as compared with a length in an axial direction of the surgery insert assisting instrument.

### (Additional feature 7)

A living tissue resection method, comprising:
an insertion step of, in a state in which a detention snare is removably coupled with a snare coupling section of a snare manipulating instrument, inserting the detention snare into a body;
a ligation step of, in a state in which a ligation target is inserted into a loop section of a snare wire of the detention snare, ligating the ligation target in the loop section of the snare wire by manipulating the loop section of the snare wire to be throttled in a throttle direction;
a detention step of, after ligating the living tissue of the ligation target, removing and retaining the detention snare from the snare coupling section of the snare manipulating instrument; and
a living tissue resection step of, after a resection surgical instrument has been disposed at an upper portion of a ligated site at a root site of the living tissue of the ligation target ligated by means of the detention snare, and in a state in which the resection surgical instrument is moved while a throttle member for throttling the loop section of the snare wire is used as a guide, and then, a predetermined cutaway is left between an upper resection face of the ligated site and the ligated site, resecting the upper portion of the ligated site.

### (Conventional technique of additional feature 5)

There is a system for throttling a root side by a detention snare or T-BAR and the like, and then, resecting an upper part thereof by the snare, thereby carrying out tissue resection without unexpected bleeding or perforation.

### (Problem to be solved by additional feature 5)

Conventionally, when a tissue is resected by means of a resection snare, the snare slips to a root side due to throttling, whereby a resection cross section is in the vicinity immediately above the detention snare or T-BAR, and a sufficient margin has not been successfully provided between a throttle position and a resection face.

### (Object of additional feature 5)

It is an object of the present invention to provide a system of a detention snare or T-BAR enabling resection by a snare while reliably providing a sufficient margin.

### Industrial Applicability

The present invention is effective in a technical field of manufacturing and using a medical ligation apparatus inserted into a body, the medical ligation apparatus retaining a ligation target in the body in a state in which the target is ligated by means of a detention snare.

## Claims

1. A medical ligation apparatus comprising:
a detention snare inserted into a body and retained therein; and
a snare manipulating instrument which manipulates the detention snare,
the detention snare having:
a snare wire having a distal end and a proximal end, the snare wire having formed thereat a ligation loop section which is expandable in a loop shape at the distal end side thereof;
a coupling ring provided at a proximal end side of the snare wire; and
a ring shaped throttle member into which the proximal end side of the snare wire is inserted in a press-fitted state,
the snare manipulating instrument having:
an elongated insert section having a distal end and a proximal end and inserted into a body; and
a snare coupling section provided at a distal end of the insert section, the snare coupling section having the coupling ring removably coupled therewith,
wherein the medical ligation apparatus comprises:
ligation means for ligating a ligation target in a loop section of the snare wire by manipulating the loop section of the snare wire to be throttled in a throttle direction by manipulating the throttle member to be pushed out to a distal end side of the snare wire in a state in which the detention snare is coupled with the snare coupling section and a living tissue of a ligation target is inserted into the loop section of the snare wire; and
living tissue cut-in preventing means which is disposed in at least one of a distal end side and a rear end side of the loop section of the snare wire, and when the living tissue of the ligation target is ligated by means of the snare wire, the means comprising a contact face on which a contact area with the living tissue is greater than a contact area of the snare wire and the living tissue.

2. The medical ligation apparatus according to claim 1, **characterized in that** the living tissue cut-in preventing means has slip preventing means with a living tissue of a ligation target on the contact face.

3. The medical ligation apparatus according to claim 1, **characterized in that** the living tissue cut-in preventing means is provided forwardly and backwardly of a loop section of the snare wire, respectively.

4. The medical ligation apparatus according to claim 1, **characterized in that** the living tissue cut-in preventing means has a cutaway setting section for, when resecting an upper side of a ligated site at a root side of a living tissue of a ligation target ligated by the detention snare, setting a predetermined cutaway between a resection face at the upper side of the ligated site and the ligated site.

5. The medical ligation apparatus according to claim 4, **characterized in that** the cutaway setting section is formed of a clip member mounted on the ligated site of the living tissue, after the living tissue is ligated by throttling the loop section by the throttle member.

6. A surgical instrument **characterized by** comprising:
a surgery insert assisting instrument **characterized by** comprising: an opening section having formed at an insert section inserted into a cavity in a body at least an endoscope insert channel, a ligation channel into which a ligation surgical instrument for inserting a ligation instrument for ligation of a living tissue is inserted, and a resection channel into which a resection surgical instrument for resecting a ligated site of the living tissue ligated by the ligation instrument is inserted, the opening section being disposed at a distal end of the insert section to insert the living tissue of the ligation target into one side of a distal end outer periphery face of the insert section; and a work space section formed at the other end of the distal end outer periphery face to manipulate the living tissue of the ligation target to be pulled up from the opening section;
the ligation surgical instrument inserted into the ligation channel and set to be disposed at a position surrounding the periphery of the opening section;
an endoscope inserted into the work space section through the endoscope insert channel;
a grip surgical instrument having provided thereat a grip section which grips the living tissue at a distal end of an insert section inserted into the work space section through the endoscope insert channel;
tissue pulling up means for manipulating the living tissue of the ligation target to be pulled up through an inside of the opening section by means of a curve manipulation of the endoscope in a state in which the living tissue is gripped by means of the grip section of the grip surgical instrument;
ligation means for ligating by means of the ligation surgical instrument the living tissue of the ligation target pulled up by the tissue pulling up means; and
a resection surgical instrument inserted into the resection channel, the resection surgical instrument resecting the ligated site of the living tissue ligated by the ligation means,
wherein a shape of the opening section of the surgery insert assisting instrument is set so as to reduce a length in a direction orthogonal to the axial direction as compared with a length in an axial direction of the surgery insert assisting instrument.

7. A living tissue resection method, **characterized by** comprising:
an insertion step of, in a state in which a detention snare is removably coupled with a snare coupling section of a snare manipulating instrument, inserting the detention snare into a body;
a ligation step of, in a state in which a ligation target is inserted into a loop section of a snare wire of the detention snare, ligating the ligation target in the loop section of the snare wire by manipulating the loop section of the snare wire to be throttled in a throttle direction;
a detention step of, after ligating the living tissue of the ligation target, removing and retaining the detention snare from the snare coupling section of the snare manipulating instrument; and
a living tissue resection step of, after a resection surgical instrument has been disposed at an upper portion of a ligated site at a root site of the living tissue of the ligation target ligated by means of the detention snare, and in a state in which the resection surgical instrument is moved while a throttle member for throttling the loop section of the snare wire is used as a guide, and then, a predetermined cutaway is left between an upper resection face of the ligated site and the ligated site, resecting the upper portion of the ligated site.
